# EUROPEAN PATENT SPECIFICATION

(11) **EP 4 151 215 B1**
(45) Date of publication and mention of the grant of the patent: **02.10.2024**
(21) Application number: 21804923.7
(22) Date of filing: 26.04.2021
(51) Int. Cl.: A61K 31/496, A61K 9/70, A61K 47/32, A61P 25/18

(54) **BLONANSERIN-CONTAINING PATCH AND PRODUCTION METHOD THEREFOR**
BLONANSERINHALTIGES PFLASTER UND HERSTELLUNGSVERFAHREN DAFÜR
PATCH CONTENANT DE LA BLONANSÉRINE ET SON PROCÉDÉ DE PRODUCTION

(30) Priority: 14.05.2020 JP 2020085385
(43) Date of publication of application: 22.03.2023
(73) Proprietor: Kaneka Corporation, Osaka-shi, Osaka 530-8288 (JP)
(72) Inventor: OGINO, Hiroyuki, Takasago-shi, Hyogo 676-8688 (JP); MATSUO, Atsuki, Takasago-shi, Hyogo 676-8688 (JP); GOTO, Masaoki, Takasago-shi, Hyogo 676-8688 (JP)
(74) Representative: Hoffmann Eitle
(86) International application number: PCT/JP2021/016641
(87) International publication number: WO 2021/230064

(56) References cited:
- WO-A1-2007/142295
- WO-A1-2012/105622
- WO-A1-2012/105624
- JP-A- 2016 003 196
- US-A1- 2009 169 605
- US-A1- 2014 023 695
- US-A1- 2019 000 774

## Description

### Technical Field

The present invention relates to a blonanserin-containing patch and a production method thereof. More specifically, the present invention relates to a blonanserin-containing patch that does not easily precipitate crystals and a production method thereof.

### Background Art

In the treatment of schizophrenia, the relapse or recurrence due to poor medication adherence of therapeutic agents (oral formulations) for schizophrenia have raised problems. Lonasen (Registered Trademark) tape, which was put on the market in September, 2019, is a schizophrenia-treating patch that contains blonanserin as an active ingredient, and it is expected that proposing a new dosage form in the treatment of schizophrenia, in which only oral medicines have been used so far, increases the options of the therapeutic agents and leads to improvement of the medication adherence for patients.

The Lonasen (Registered Trademark) tape has a low drug utilization rate (NPL 1), and the size of a formulation needed to be large in order to absorb a sufficient amount of the drug from the skin and to obtain a blood level of the drug necessary for the treatment.

In addition, blonanserin had problems in terms of storage stability, because blonanserin has a high crystallinity and may crystallize on the surface of an adhesive layer during storage of a blonanserin-containing patch.

Therefore, it is desirable to provide a blonanserin-containing patch having a high skin permeability, and also be resistant to crystallization and has excellent storage stability, and to provide a production method thereof.

US2014/023695A1 discloses a patch preparation containing a support and an adhesive layer formed on one surface of the support, wherein the adhesive layer contains blonanserin. US2009/169605A1 discloses a tape preparation containing blonanserin. US2019/000774A1 relates to a transdermal absorption preparation comprising a support and a drug-containing adhesive layer.

### Citation List

### Non-Patent Literature

NPL 1: Medicine Interview Form Lonasen (Registered Trademark) tape (published in June, 2019, 1st edition)

### Summary of Invention

### Technical Problem

An object of the present invention is to solve the existing problems, and to achieve the following object. That is, the object of the present invention is to provide a blonanserin-containing patch that is resistant to crystallization, has excellent storage stability, and has a high skin permeability, and a production method thereof.

### Solution to Problem

As a result of diligent studies in order to solve the problem, the present inventors found that a patch including: a support; and an adhesive layer on the support, the adhesive layer including blonanserin or a salt thereof, a thermoplastic elastomer, and a higher fatty acid ester makes it possible to provide a blonanserin-containing patch that is resistant to crystallization, has excellent storage stability, and has a high skin permeability, and completed the present invention.

The present invention is based on the finding obtained by the present inventors, and means for solving the aforementioned problem are as follows. That is,
<1> A patch including:
   a support; and
   an adhesive layer on the support,
   wherein the adhesive layer includes blonanserin or a salt thereof, a thermoplastic elastomer, and a higher fatty acid ester.
<2> A production method of the patch according to <1>, the method including:
   laminating the support and the adhesive layer.

### Advantageous Effects of Invention

According to the present invention, it is possible to solve the existing problems, to achieve the above object, and to provide a blonanserin-containing patch, which is suppressed in crystallization and has a high skin permeability, and a production method thereof.

### Description of Embodiments

### (Patch)

The patch includes a support and an adhesive layer on the support, and may further include other components.

### <Support>

The support is not particularly limited and may be appropriately selected in accordance with the intended purpose. For example, those that can be commonly used in adhesive sheets for skin patching or transdermal absorption formulations can be used.

The material of the support is not particularly limited and may be appropriately selected in accordance with the intended purpose. Examples thereof include: polyester such as polyethylene terephthalate or the like; polyolefin such as polyethylene, polypropylene, or the like; polyurethane; an ethylene-vinyl acetate copolymer; and polyvinyl chloride.

The structure of the support may be a single layer structure or may be a multilayer structure. Moreover, the support may be a knit fabric, a nonwoven fabric, a film, an expanded body, a porous material, a material having a mesh structure, a sheet-like material, a plate-like material, or the like.

In addition, in order to suppress accumulation of static electricity in the support, the woven fabric, the nonwoven fabric, the film, or the like may include an antistatic agent. Moreover, in order to obtain a good anchoring property with the adhesive layer, a laminated body of a nonwoven fabric or a woven fabric, or of these and a film can be used as the support.

The thickness of the support is not particularly limited and may be appropriately selected in accordance with the intended purpose . When the support is a film, its thickness is preferably 10 µm or more and 100 µm or less, and more preferably 15 µm or more and 50 µm or less. When the support is a woven fabric, a nonwoven fabric, or a porous sheet such as an expanded support or the like, its thickness is preferably 50 µm or more and 2,000 µm or less, and more preferably 100 µm or more and 1,000 µm or less.

### <Adhesive layer>

The adhesive layer includes (a) blonanserin or a salt thereof, (b) a thermoplastic elastomer, and (c) a higher fatty acid ester, and may further include other components.

### (a) Blonanserin or salt thereof

The "blonanserin", which is a compound represented by a chemical name of 2-(4-ethyl-1-piperazinyl)-4-(4-fluorophenyl)-5,6,7,8,9,10-hexahy droxycycloocta[b]pyridine, is classified into SDA (serotonin-dopamine antagonist), and is commercially available as an antipsychotic (atypical antipsychotic).

The blonanserin may be a free base or may be a pharmaceutically acceptable salt, and is not particularly limited.

The pharmaceutically acceptable salt is not particularly limited and may be appropriately selected in accordance with the intended purpose. Examples thereof include pharmaceutically acceptable acid addition salts, which may be inorganic salts or may be organic salts.

The pharmaceutically acceptable salt may be used alone or in combination. Moreover, the free base and the pharmaceutically acceptable salt may be used in a mixture.

The inorganic salt is not particularly limited and may be appropriately selected in accordance with the intended purpose. Examples thereof include hydrochlorides, hydrobromides, nitrates, sulfates, and phosphates.

Examples of the organic salts include formates, acetates, trifluoroacetates, propionates, lactates, tartrates, oxalates, fumarates, maleates, citrates, malonates, and methanesulfonates. In terms of easily availability, a free base or a hydrochloride is preferable. In terms of skin permeability, a free base is preferably used.

The amount of blonanserin or the salt thereof in the adhesive layer; i.e., a ratio of the blonanserin or the salt thereof relative to the total amount 100% by mass of the constituent components of the adhesive layer is not particularly limited and may be appropriately selected in accordance with the intended purpose. In terms of ensuring dispersibility in the adhesive layer and good skin permeability, the lower limit thereof is preferably 0.5% by mass or more, more preferably 0.75% by mass or more, still more preferably 1% by mass or more, and particularly preferably 1.5% by mass or more, and the upper limit thereof is preferably 30% by mass or less, more preferably 25% by mass or less, still more preferably 20% by mass or less, and particularly preferably 15% by mass or less.

### (b) Thermoplastic elastomer

The "thermoplastic elastomer" according to the present disclosure is a thermoplastic elastomer, which exhibits such flowability that the thermoplastic elastomer softens when heat is applied thereto and exhibits such thermoplasticity that the softened thermoplastic elastomer returns to the rubber-like elastic body when it is cooled.

According to the invention as defined in the claims, the thermoplastic elastomer is a styrene-based block copolymer. In particular, in order to achieve both sufficient adhesiveness to the skin and low skin irritation, styrene-based block copolymers are preferable.

The styrene-based block copolymer is not particularly limited and may be appropriately selected in accordance with the intended purpose. Examples thereof include styrene-butadiene block copolymers, styrene-butadiene-styrene block copolymers, styrene-isoprene block copolymers, styrene-isoprene-styrene block copolymers, styrene-ethylene/butylene block copolymers, styrene-ethylene/butylene-styrene block copolymers, styrene-ethylene/propylene block copolymers, styrene-ethylene/propylene-styrene block copolymers, styrene-isobutylene block copolymers, and styrene-isobutylene-styrene block copolymers. Note that, the "ethylene/butylene" represents a copolymer block of ethylene and butylene, and the "ethylene/propylene" represents a copolymer block of ethylene and propylene. These styrene-based block copolymers may be used alone or in combination.

Among the styrene-based block copolymers, one kind or two kinds or more styrene-based block copolymers, which are selected from the group consisting of the styrene-isoprene-styrene block copolymers and the styrene-isoprene block copolymers, are preferable, and a mixture of a styrene-isoprene block copolymer and a styrene-isoprene-styrene block copolymer is particularly preferable. The reason for this is because both sufficient adhesiveness of the adhesive layer to the skin and prevention of adhesive residues due to improvement of a cohesive force are achieved, as well as from the viewpoint of availability and handling property.

The lower limit of the ratio of the styrene·isoprene block copolymer in the mixture is not particularly limited and may be appropriately selected in accordance with the intended purpose. The lower limit thereof is preferably 10% by mass or more, more preferably 15% by mass or more, still more preferably 20% by mass or more, particularly preferably 40% by mass or more, and most preferably 50% by mass or more.

The upper limit of the ratio of the styrene·isoprene block copolymer in the mixture is not particularly limited and may be appropriately selected in accordance with the intended purpose. The upper limit thereof is preferably 95% by mass or less, more preferably 90% by mass or less, still more preferably 85% by mass or less, and particularly preferably 80% by mass or less.

The amount of styrene in the styrene·isoprene·styrene block copolymer is not particularly limited and may be appropriately selected in accordance with the intended purpose. The amount of styrene in the copolymer is preferably 5% by mass or more and 60% by mass or less, and more preferably 10% by mass or more and 50% by mass or less.

The molecular weight of the styrene·isoprene·styrene block copolymer is not particularly limited and may be appropriately selected in accordance with the intended purpose. The weight average molecular weight, which is measured through gel permeation chromatography (GPC), is preferably 20,000 or more and 500,000 or less, and more preferably 30,000 or more and 300,000 or less.

The amount of styrene in the styrene·isoprene block copolymer is not particularly limited and may be appropriately selected in accordance with the intended purpose. The amount of styrene in the copolymer is preferably 5% by mass or more and 50% by mass or less, and more preferably 10% by mass or more and 40% by mass or less.

The molecular weight of the styrene·isoprene block copolymer is not particularly limited and may be appropriately selected in accordance with the intended purpose. The weight average molecular weight, which is measured through GPC, is preferably 10,000 or more and 500,000 or less, and more preferably 20,000 or more and 300,000 or less.

The viscosity of the styrene-based block copolymer is not particularly limited and may be appropriately selected in accordance with the intended purpose. To improve a balance of adhesive substances, the lower limit of a solution viscosity of a 25% by mass toluene solution of the styrene-based block copolymer at 25°C is preferably 500 mPa·s or more, and more preferably 900 mPa·s or more, and the upper limit thereof is preferably 2,000 mPa·s or less, and more preferably 1, 800 mPa·s or less. Note that the "solution viscosity of the 25% by mass toluene solution at 25°C" is a value measured based on a method of measuring the viscosity of a styrene·isoprene·styrene block copolymer described on page 395 of "Pharmaceutical Excipients Standard 2013" (published by Yakuji Nippo Ltd.).

As each of the styrene·isoprene·styrene block copolymer and the styrene·isoprene block copolymer, a copolymer produced by a traditional method can be used. Moreover, as each of the styrene·isoprene·styrene block copolymer and the styrene·isoprene block copolymer, a commercially available product that satisfies the aforementioned characteristics can be used. In addition, a mixture of the styrene·isoprene·styrene block copolymer and the styrene·isoprene block copolymer is also commercially available, and a commercially available product of the mixture, which satisfies the aforementioned characteristics and is obtained by mixing the styrene·isoprene·styrene block copolymer and the styrene·isoprene block copolymer at the aforementioned mixing ratio, can be suitably used.

Examples of commercially available styrene-based block copolymers include: "KRATON (Registered Trademark) D1111", "KRATON (Registered Trademark) D1163", "KRATON (Registered Trademark) D1113", and "KRATON (Registered Trademark) D1119", all of which are manufactured by KRATON POLYMERS; "JSR (Registered Trademark) SIS5229", "JSR (Registered Trademark) SIS5002", "JSR (Registered Trademark) SIS5403", and "JSR (Registered Trademark) SIS5505", all of which are manufactured by JSR; and "Quintac (Registered Trademark) 3421", "Quintac (Registered Trademark) 3433N", "Quintac (Registered Trademark) 3520", "Quintac (Registered Trademark) 3450", and "Quintac (Registered Trademark) 3270", all of which are manufactured by Zeon Corporation.

Among the above commercially available styrene-based block copolymers, in terms of the mixing ratio between the styrene·isoprene·styrene block copolymer and the styrene·isoprene block copolymer and the solution viscosity, "KRATON (Registered Trademark) D1163", "KRATON (Registered Trademark) D1113", "JSR (Registered Trademark) SIS5403", "JSR (Registered Trademark) SIS5505", "Quintac (Registered Trademark) 3433N", and "Quintac (Registered Trademark) 3520" are preferable, and "JSR (Registered Trademark) SIS5505" and/or "Quintac (Registered Trademark) 3520" are particularly preferable.

The amount of the thermoplastic elastomer in the adhesive layer; i.e., the ratio of the thermoplastic elastomer relative to the total amount 100% by mass of the constituent components of the adhesive layer is not particularly limited and may be appropriately selected in accordance with the intended purpose. The lower limit thereof is preferably 20% by mass or more, more preferably 25% by mass or more, and still more preferably 30% by mass or more. The upper limit thereof is preferably 70% by mass or less, more preferably 65% by mass or less, and still more preferably 60% by mass or less. When the ratio is 20% by mass or more, the shape of the adhesive layer can be securely maintained. When the ratio is 70% by mass or less, adhesiveness of the adhesive layer to the skin is more securely exhibited.

### (c) Higher fatty acid ester

The "higher fatty acid ester" in the present invention is a compound obtained by binding a carboxyl group of a higher fatty acid and an aliphatic alcohol via an ester bond. The higher fatty acid ester has an effect of appropriately plasticizing the thermoplastic elastomer, and contributes to impartment of adhesiveness. The higher fatty acid ester exhibits appropriate affinity with blonanserin, and contributes to improvement of solubility of blonanserin in the adhesive layer and prevention of crystallization.

The higher fatty acid constituting the higher fatty acid ester may be a straight chain higher fatty acid or a branched chain higher fatty acid. The higher fatty acid may be saturated or unsaturated. In terms of the plasticizing effect and the thermal stability of the thermoplastic elastomer, a saturated fatty acid is preferable. The number of carbon atoms of the higher fatty acid is preferably 12 or more, more preferably 14 or more, and still more preferably 16 or more, and is preferably 30 or less, more preferably 24 or less, and still more preferably 20 or less.

Examples of saturated higher fatty acids include capric acid (a number of carbon atoms is 10), lauric acid (a number of carbon atoms is 12), myristic acid (a number of carbon atoms is 14), palmitic acid (a number of carbon atoms is 16), stearic acid (a number of carbon atoms is 18), isostearic acid (a number of carbon atoms is 18), arachidic acid (a number of carbon atoms is 20), behenic acid (a number of carbon atoms is 22), lignoceric acid (a number of carbon atoms is 24), cerotic acid (a number of carbon atoms is 26), montanic acid (a number of carbon atoms is 28), and melissic acid (a number of carbon atoms is 30) . Among them, myristic acid, palmitic acid, and stearic acid are preferable.

Examples of unsaturated higher fatty acids include palmitoleic acid (a number of carbon atoms is 16), oleic acid (a number of carbon atoms is 18), linoleic acid (a number of carbon atoms is 18), (9,12,15)-linolenic acid (a number of carbon atoms is 18), (6,9,12)-linolenic acid (a number of carbon atoms is 18), and eleostearic acid (a number of carbon atoms is 18) . Among them, oleic acid and linoleic acid are preferable.

Examples of aliphatic alcohols constituting the higher fatty acid ester include saturated or unsaturated aliphatic alcohols having 1 or more and 30 or less carbon atoms, such as methanol, ethanol, propanol, isopropanol, butanol, hexanol, heptanol, octanol, decanol, cetanol, myristyl alcohol, hexyldecanol, oleyl alcohol, octyldodecanol, and the like. The number of carbon atoms of the aliphatic alcohol corresponds to the number of carbon atoms of the ester portion of the higher fatty acid ester. The number of carbon atoms of the aliphatic alcohol is preferably 12 or more and 30 or less. When the number of carbon atoms is 12 or more, a plasticizing effect due to the aliphatic alcohol is more securely exhibited. On the other hand, when the number of carbon atoms is 30 or less, the solubility of blonanserin in the adhesive layer can be sufficiently ensured.

Suitable specific examples of the higher fatty acid esters include: myristates such as isopropyl myristate, ethyl myristate, octyldodecyl myristate, and the like; palmitates such as isopropyl palmitate, ethyl palmitate, and the like; stearates such as isopropyl stearate and the like; oleates such as decyl oleate, octyldodecyl oleate, oleyl oleate, and the like; and linoleates such as ethyl linoleate and the like. Among them, octyldodecyl myristate is particularly preferable in terms of plasticity and solubility of blonanserin.

The ratio of the higher fatty acid ester relative to 100 parts by mass of the thermoplastic elastomer in the adhesive layer is preferably 25 parts by mass or more and 300 parts by mass or less. When the ratio is 25 parts by mass or more, good adhesiveness of the adhesive layer and solubility of blonanserin are more securely exhibited. When the ratio is 300 parts by mass or less, the shape of the adhesive layer can be more securely maintained. The ratio is more preferably 30 parts by mass or more and 200 parts by mass or less. Because of the same reasons, the amount of the higher fatty acid ester in the adhesive layer of the patch according to the present disclosure is preferably 10% by mass or more, more preferably 15% by mass or more, and still more preferably 20% by mass or more, and is preferably 75% by mass or less, and more preferably 70% by mass or less, and still more preferably 65% by mass or less.

### -Other components-

The adhesive layer may include, as other components, for example, (d) polyisobutylene, (e) an aliphatic dicarboxylic acid ester, (f) glycerin monoether, (g1) a fatty acid monoester of polyhydric alcohol, (g2) a higher alcohol, (g3) an alcohol-based solvent, (g4) an amide-based solvent, (g5) an ester-based solvent, (g6) a liquid organic acid, (g7) a carboxylate, (g8) lactone, (g9) a surfactant, (g10) a filler, (g11) an inhibitor of crystallization, and (h) a tackifier, if necessary.

### (d) Polyisobutylene

In order to adjust adhesive physical properties, polyisobutylene can be added.

The "polyisobutylene" used in the adhesive layer of the present invention is a polymer of isobutylene and is an elastic rubber-like semisolid or viscous substance. In the present invention, the "polyisobutylene" is added in order to impart sufficient adhesiveness to the skin.

As the polyisobutylene, a low-molecular-weight polyisobutylene having a viscosity average molecular weight of from 30, 000 to 100,000, a middle-molecular-weight polyisobutylene having a viscosity average molecular weight of from 100,000 to 500,000, and a high-molecular-weight polyisobutylene having a viscosity average molecular weight of from 500,000 to 5,000,000 can be each used alone or can be used as a mixture. In particular, mixing the low-molecular-weight polyisobutylene and the high-molecular-weight polyisobutylene or using the middle-molecular-weight polyisobutylene alone is preferable in order to maintain balance between low skin irritation and high adhesiveness to the skin.

As the polyisobutylene, a polymer of isobutylene produced by a traditional method can be used. In particular, in the adhesive layer of the present invention for skin patch, those conforming to the standards of Japanese pharmaceutical excipients or those conforming to the standards stipulated in, for example United States Pharmacopeia can be preferably used. Moreover, as polyisobutylene, a commercially available product satisfying the respective viscosity average molecular weights described above can be used.

Examples of the commercially available low-molecular-weight polyisobutylenes include "Oppanol (Registered Trademark) B10SFN", "Oppanol (Registered Trademark) B10N", "Oppanol (Registered Trademark) B12SFN", "Oppanol (Registered Trademark) B15SFN", and "Oppanol (Registered Trademark) B15N", all of which are manufactured by BASF. Examples of the commercially available products of the middle-molecular-weight polyisobutylene include "Oppanol (Registered Trademark) N50SF" and "Oppanol (Registered Trademark) N50", both of which are manufactured by BASF. Examples of the commercially available products of the high-molecular-weight polyisobutylene include "Oppanol (Registered Trademark) N80", "Oppanol (Registered Trademark) N100", and "Oppanol (Registered Trademark) N150", all of which are manufactured by BASF.

Among the commercially available polyisobutylenes, in terms of the solubility at the time of preparing a coating liquid or the balance of adhesive physical properties of the obtained patch, "Oppanol (Registered Trademark) B15SFN" and "Oppanol (Registered Trademark) B15N" having a viscosity average molecular weight of from 50,000 to 100,000 are particularly preferable as the low-molecular-weight polyisobutylene, "Oppanol (Registered Trademark) N50SF" and "Oppanol (Registered Trademark) N50" are particularly preferable as the middle-molecular-weight polyisobutylene, and "Oppanol (Registered Trademark) N80" is particularly preferable as the high-molecular-weight polyisobutylene.

The amount of polyisobutylene in the adhesive layer is not particularly limited and may be appropriately selected in accordance with the intended purpose. When the amount of polyisobutylene in the adhesive layer is too small, enhancement of adhesiveness to the skin may be insufficient. When the amount thereof is too large, problems such as deterioration of skin irritation due to excessively enhanced adhesiveness to the skin, adhesive residues at the time of removal, poor solubility of pharmacological agents, and the like may be caused. Therefore, the lower limit of the amount of polyisobutylene in the adhesive layer is 0.1 parts by weight or more, preferably 0.3 parts by weight or more, more preferably 0.5 parts by weight or more, and still more preferably 1 part by weight or more, relative to 100 parts by weight of the thermoplastic elastomer. The upper limit of the amount of polyisobutylene in the adhesive layer is 300 parts by weight or less, preferably 100 parts by weight or less, more preferably 50 parts by weight or less, and still more preferably 30 parts by weight or less, relative to 100 parts by weight of the thermoplastic elastomer.

As a particularly suitable aspect, the amount of polyisobutylene in the adhesive layer can be from 0.1% by mass to 50% by mass, more preferably from 0.2% by mass to 40% by mass, still more preferably from 0.3% by mass to 30% by mass, and particularly preferably from 0.5% by mass to 25% by mass.

### (e) Aliphatic dicarboxylic acid ester

The aliphatic dicarboxylic acid ester is not particularly limited and may be appropriately selected in accordance with the intended purpose. Examples thereof include diesters of dicarboxylic acid having 2 or more and 12 or less carbon atoms and monohydric aliphatic alcohol having 1 or more and 20 or less carbon atoms, which are liquid at ordinary temperature, such as liquid adipic acid diesters at ordinary temperature including diethyl adipate, diisopropyl adipate, diisobutyl adipate, and the like; and liquid sebacic acid diesters at ordinary temperature including diethyl sebacate, diisopropyl sebacate, dioctyldodecyl sebacate, and the like. Among them, in terms of improvement of solubility of drugs and an absorption promoting effect, diisopropyl adipate and diisobutyl adipate are preferable.

The lower limit of the amount of the aliphatic dicarboxylic acid ester relative to the total amount 100% by mass of the constituent components of the adhesive layer is not particularly limited and may be appropriately selected in accordance with the intended purpose. The lower limit thereof is preferably 0.2% by mass or more, more preferably 0.5% by mass or more, still more preferably 1% by mass or more, and particularly preferably 1.5% by mass or more.

The upper limit of the amount of the aliphatic dicarboxylic acid ester relative to the total amount 100% by mass of the constituent components of the adhesive layer is not particularly limited and may be appropriately selected in accordance with the intended purpose. The upper limit thereof is preferably 20% by mass or less, more preferably 10% by mass or less, still more preferably 5% by mass or less, and particularly preferably 3% by mass or less.

### (f) Glycerin monoether

The glycerin monoether is not particularly limited and may be appropriately selected in accordance with the intended purpose. Examples thereof include α-monoisostearyl glyceryl monoether.

The lower limit of the amount of the glycerin monoether relative to the total amount 100% by mass of the constituent components of the adhesive layer is not particularly limited and may be appropriately selected in accordance with the intended purpose. The lower limit thereof is preferably 0.05% by mass or more, more preferably 0.1% by mass or more, still more preferably 0.2% by mass or more, and particularly preferably 0.3% by mass or more.

The upper limit of the amount of the glycerin monoether relative to the total amount 100% by mass of the constituent components of the adhesive layer is not particularly limited and may be appropriately selected in accordance with the intended purpose. The upper limit thereof is preferably 10% by mass or less, more preferably 5% by mass or less, still more preferably 1.5% by mass or less, and particularly preferably 1.0% by mass or less.

### (g1) Fatty acid monoester of polyhydric alcohol

In the present invention, the "fatty acid monoester of polyhydric alcohol" is a compound obtained by binding one hydroxyl group of a polyhydric alcohol such as ethylene glycol, propylene glycol, glycerin, or the like with fatty acid via an ester bond. The fatty acid monoester of polyhydric alcohol contributes to improvement of the solubility of drugs and has an absorption promoting effect without significantly decreasing a cohesive force of an adhesive base material.

The polyhydric alcohol constituting the fatty acid monoester of polyhydric alcohol is not particularly limited and may be appropriately selected in accordance with the intended purpose. Examples thereof include ethylene glycol, propylene glycol, butylene glycol, and glycerin.

The fatty acid constituting the fatty acid monoester of polyhydric alcohol is not particularly limited and may be appropriately selected in accordance with the intended purpose, but a fatty acid having 8 or more and 18 or less carbon atoms is preferable. Examples thereof include capric acid, caprylic acid, myristic acid, palmitic acid, stearic acid, isostearic acid, oleic acid, and linoleic acid.

Suitable specific examples of the fatty acid monoester of polyhydric alcohol include propylene glycol monocaprylate and propylene glycol monolaurate. In order to increase the solubility of drugs and an absorption promoting effect, the amount of the fatty acid monoester of polyhydric alcohol is preferably 2% by mass or more, and more preferably 5% by mass or more, relative to the total amount of the adhesive agent components.

On the other hand, when a large amount of the fatty acid monoester of polyhydric alcohol is added, the cohesive force of the adhesive force and the adhesive force are decreased. Therefore, the amount of the fatty acid monoester of polyhydric alcohol is preferably 30% by mass or less relative to the total amount of the adhesive agent components.

### (g2) Higher alcohol

The higher alcohol is not particularly limited and may be appropriately selected in accordance with the intended purpose. Examples thereof include: higher saturated aliphatic alcohols having about 12 or more and about 20 or less carbon atoms, which are liquid at ordinary temperature, such as lauryl alcohol, isostearyl alcohol, and the like; and higher unsaturated aliphatic alcohols having about 12 or more and about 20 or less carbon atoms, which are liquid at ordinary temperature, such as oleyl alcohol and the like.

Among them, lauryl alcohol and oleyl alcohol are preferable in terms of improvement of the solubility of drugs and an absorption promoting effect.

### (g3) Alcohol-based solvent

The alcohol-based solvent is not particularly limited and may be appropriately selected in accordance with the intended purpose. Examples thereof include: ethylene glycol; propylene glycol; glycerin; 1,3-butanediol; polyhydric alcohols having a molecular weight of about 100 or more and about 600 or less, which are liquid at ordinary temperature, such as polyethylene glycol and the like; monoalkyl ethers of polyhydric alcohols such as diethylene glycol monoethyl ether, and the like; and monofatty acid esters of polyhydric alcohols such as glycerol monolinoleate, glycerol monooleate, and the like.

Among them, in terms of improvement of the solubility of drugs, ethylene glycol, propylene glycol, glycerin, and 1,3-butanediol are preferable.

### (g4) Amide-based solvent

The amide-based solvent is not particularly limited and may be appropriately selected in accordance with the intended purpose. Examples thereof include: pyrrolidone such as N-methyl-2-pyrrolidone, 2-pyrrolidone, and the like; imidazolidinone such as 1,3-dimetyl-2-imidazolidinone, and the like; N-substituted toluidine such as crotamiton, and the like; and alkanamide such as formamide, N-methylformamide, N,N-dimethylformamide, N-methylacetamide, N,N-dimetylacetamide, N-methyl propenamide, and the like.

Among the amide-based solvents, in terms of improvement of the solubility of drugs, the dispersibility, and the percutaneous absorption property, N-methy-l-2-pyrrolidone, crotamiton, N,N-dimethylformamide, and N,N-dimetylacetamide are preferable, and N-methyl-2-pyrrolidone and crotamiton are more preferable.

### (g5) Ester-based solvent

The ester-based solvent is not particularly limited and may be appropriately selected in accordance with the intended purpose. Examples thereof include diesters of dihydric alcohols and carboxylic acids, medium chain fatty acid triglyceride, esters of multivalent carboxylic acids and monohydric aliphatic alcohols, and carbonates.

The diester of the dihydric alcohol and carboxylic acid is not particularly limited and may be appropriately selected in accordance with the intended purpose. Examples thereof include diesters constituted by propylene glycol, and caprylic acid, capric acid, lauric acid, oleic acid, or the like.

The medium chain fatty acid triglyceride is triglyceride, which consists of: a fatty acid having about 6 or more and about 12 or less carbon atoms, such as caproic acid, caprylic acid, capric acid, lauric acid, or the like; and glycerin. In the present invention, caprylic acid triglyceride, a triglyceride mixture of caprylic acid and capric acid, a triglyceride mixture of caprylic acid, capric acid, and lauric acid, and the like, which are liquid at ordinary temperature, can be used. In addition, oils, which are liquid at ordinary temperature and include the above in large amounts, can also be used. Examples of the oils include olive oil, almond oil, safflower oil, soybean oil, corn oil, sesame oil, coconut oil, orange oil, ginger oil, orange peel oil, rape seed oil, castor oil, sunflower oil, cotton seed oil, and peanut oil.

Here, in the present invention, as a liquid medium chain fatty acid triglyceride at ordinary temperature or a liquid medium chain fatty acid triglyceride-including oil at ordinary temperature, a commercially available product for pharmaceutical use can also be used.

The carbonate is not particularly limited and may be appropriately selected in accordance with the intended purpose. Examples thereof include cyclic carbonates of carbonic acid and diol having 2 or more and 10 or less carbon atoms, such as ethylene carbonate, propylene carbonate, vinylene carbonate, and the like, and propylene carbonate is preferable.

Among the aforementioned ester-based solvents, medium chain fatty acid triglyceride mixtures and carbonates are preferable, triglyceride mixtures of caprylic acid and capric acid, and propylene carbonate are more preferable.

In the present invention, as the alcohol-based solvent, the amide-based solvent, and the ester-based solvent, one kind or two kinds or more selected from the aforementioned compounds can be used if necessary.

The amount of these solvents is not particularly limited and may be appropriately selected in accordance with the intended purpose . The ratio thereof relative to the total amount of the adhesive layer is preferably 0.1% by mass or more and 20% by mass or less, and more preferably 0.5% by mass or more and 15% by mass or less.

### (g6) Liquid organic acid

The liquid organic acid is not particularly limited and may be appropriately selected in accordance with the intended purpose. Examples thereof include: aliphatic monocarbonic acids such as acetic acid, propionic acid, butyric acid, valeric acid, isovaleric acid, caproic acid, enanthic acid (heptanoic acid), caprylic acid, pelargonic acid (nonanoic acid), isostearic acid, levulinic acid, and the like; aliphatic unsaturated monocarbonic acids such as oleic acid, linoleic acid, arachidonic acid, docosahexaenoic acid, and the like; alkoxy group-substituted carboxylic acids that are liquid, such as methoxyacetic acid and the like; and sulfonic acids such as methanesulfonic acid and the like.

These liquid organic acids have a function of aiding dissolution of a basic drug such as blonanserin, and can contain the basic drug in the adhesive layer at a high level. In addition, these liquid organic acids can also improve dispersibility, and further have an effect of improving a percutaneous absorption property. From these viewpoints, among these liquid organic acids, levulinic acid, oleic acid, and isostearic acid are preferable.

In the present invention, one kind or two kinds or more selected from the liquid organic acids can be contained if necessary.

The amount of the liquid organic acid is not particularly limited and may be appropriately selected in accordance with the intended purpose. The ratio thereof relative to the total amount of the adhesive layer is preferably 0.1% by mass or more and 20% by mass or less, and more preferably 0.5% by mass or more and 15% by mass or less.

### (g7) Carboxylate

The carboxylate is not particularly limited and may be appropriately selected in accordance with the intended purpose. Examples thereof include salts of aliphatic monocarbonic acids, alicyclic monocarbonic acids, aliphatic dicarboxylic acids, and the like.

The aliphatic monocarbonic acid is not particularly limited and may be appropriately selected in accordance with the intended purpose . Examples thereof include: fatty acids having 2 or more and 7 or less carbon atoms, such as acetic acid, butyric acid, hexanoic acid, and the like; fatty acids having 8 or more and 11 or less carbon atoms, such as octanoic acid, decanoic acid, and the like; fatty acids having 12 or more carbon atoms, such as lauric acid, myristic acid, stearic acid, isostearic acid, oleic acid, and the like; hydroxymonocarboxylic acids, such as glycolic acid, lactic acid, 3-hydroxybutyric acid, mandelic acid, and the like; alkoxy group-substituted monocarbonic acids, such as methoxyacetic acid; and ketomonocarbonic acids such as levulinic acid and the like.

The alicyclic monocarbonic acid is not particularly limited and may be appropriately selected in accordance with the intended purpose . Examples thereof include alicyclic monocarbonic acids having 6 or more and 8 or less carbon atoms such as cyclohexanecarboxylic acid and the like.

The aliphatic dicarboxylic acid is not particularly limited and may be appropriately selected in accordance with the intended purpose . Examples thereof include sebacic acid, adipic acid, malic acid, maleic acid, and fumaric acid.

Preferable examples of carboxylic acids include fatty acids having 12 or more carbon atoms and hydroxymonocarboxylic acids, including, for example, myristic acid, stearic acid, isostearic acid, and oleic acid. More preferable examples thereof include lauric acid and oleic acid.

The salts of the carboxylic acids are not particularly limited and may be appropriately selected in accordance with the intended purpose. Examples thereof include: salts of alkali metals such as sodium salts, potassium salts, and the like; salts of alkali earth metals such as calcium salts and the like; and amine salts. However, sodium salts are preferable in terms of easy availability and an effect of improving the percutaneous absorption property.

### (g8) Lactone

The lactone is not particularly limited and may be appropriately selected in accordance with the intended purpose. Examples thereof include 5-membered ring lactones such as ascorbic acid, isoascorbic acid, and the like.

In the patch of the present invention, preferable examples of carboxylates or lactones include sodium oleate, sodium lactate, and ascorbic acid or isoascorbic acid in consideration of an effect of improving the stability of pharmacological agents and an effect of improving the percutaneous absorption property.

When the patch of the present invention includes carboxylate or lactone, the amount of carboxylate or lactone in the adhesive layer is not particularly limited and may be appropriately selected in accordance with the intended purpose. The amount thereof is preferably 0.1 moles or more and 5 moles or less, and more preferably 0.2 moles or more and 3 moles or less, relative to 1 mole of a drug. When the addition amount relative to 1 mole of the drug is less than 0.1 moles, a sufficient effect of improving the percutaneous absorption property may not be obtained. When the addition amount relative to 1 mole of the drug is more than 5 moles, the physical properties of formulations, such as adhesive property, may be deteriorated.

### (g9) Surfactant

The surfactant is not particularly limited and may be appropriately selected in accordance with the intended purpose. Examples thereof include: nonionic surfactants, such as polyoxyethylene fatty acid esters (e.g., polyoxyethylene monolaurate), polyoxyethylene sorbit fatty acid esters (e.g., polyoxyethylene sorbit tetraoleate), polyoxyethylene sorbitan fatty acid esters (e.g., polyoxyethylene sorbitan monooleate, polyoxyethylene sorbitan monolaurate, and polyoxyethylene sorbitan monopalmitate), sorbitan fatty acid esters (e.g., sorbitan monolaurate, sorbitan monooleate, sorbitan sesquioleate, and sorbitan trioleate), glycerin fatty acid esters (e.g., glycerin monooleate, polyoxyethylene castor oil derivatives, and polyoxyethylene hydrogenated castor oil), polyoxyethylene higher aliphatic alcohol ethers (e.g., polyoxyethylene lauryl ether and polyoxyethylene oleyl ether), polyoxyethylene alkyl phenyl ethers (e.g, polyoxyethylene nonyl phenyl ether), polyoxyethylene alkyl amino ethers (e.g., polyoxyethylene laurylamine and polyoxyethylene oleylamine), and polyoxyethylene-polyoxypropylene copolymers (e.g., Pluronic (Registered Trademark) L-31 and Pluronic (Registered Trademark)L-44); anionic surfactants such as sodium alkylsulfates (e.g., sodium lauryl sulfate); cationic surfactants such as alkyltrimethylammonium salts and alkyldimethylammonium salts; and amphoteric surfactants such as alkyldimethyl amine oxide and alkylcarboxybetaine. One kind or two or more kinds selected from the above can be used.

Among the surfactants, in order to increase the percutaneous absorption property, a liquid nonionic surfactant at ordinary temperature is preferable, a liquid sorbitan fatty acid ester at ordinary temperature is more preferable, and sorbitan monolaurate is particularly preferable.

When the patch of the present invention includes a surfactant, the amount of the surfactant in the adhesive layer is not particularly limited and may be appropriately selected in accordance with the intended purpose. The amount thereof is preferably 0.01% by mass or more and 10% by mass or less, and more preferably 0.1% by mass or more and 5% by mass or less.

### (g10) Filler

In order to control the flexibility of the adhesive layer, a filler can be included.

The filler is not particularly limited and may be appropriately selected in accordance with the intended purpose. Examples thereof include: silicon compounds such as silicic anhydride, light anhydrous silicic acid, hydrous silicic acid, and the like; cellulose derivatives, such as ethyl cellulose, methyl cellulose, hydroxypropylcellulose, hydroxypropyl methylcellulose, and the like; water-soluble polymers such as polyvinyl alcohol and the like; aluminum compounds, such as dried aluminum hydroxide gel, hydrous aluminum silicate, and the like; kaolin; and titanium oxide. The filler may be used alone or in combination.

The amount of the filler is not particularly limited and may be appropriately selected in accordance with the intended purpose. The filler can be included within such a range that can maintain a high skin permeability and sufficient cohesive force and adhesive force as the patch. Among them, the amount thereof is preferably 10% by mass or less, more preferably 5% by mass or less, and most preferably 2% by mass or less, relative to the total amount of the adhesive components.

### (g11) Inhibitor of crystallization

In order to suppress crystallization of a drug in the adhesive layer, an inhibitor of crystallization can be contained.

The inhibitor of crystallization is not particularly limited and may be appropriately selected in accordance with the intended purpose. Examples thereof include polyvinylpyrrolidone, vinyl acetate-vinylpyrrolidone copolymers, polyvinylcaprolactam-polyvinyl acetate-polyethylene glycol graft copolymers, aminoalkyl methacrylate copolymers, methacrylic acid copolymers, and ammonioalkyl methacrylate copolymers. The inhibitor of crystallization may be used alone or in combination.

The amount of the inhibitor of crystallization is not particularly limited and may be appropriately selected in accordance with the intended purpose, and can be contained within such a range that can maintain the adhesive force as the patch. Among them, the amount of the inhibitor of crystallization is preferably 0.01% by mass or more and 10% by mass or less, and more preferably 0.1% by mass or more and 5% by mass or less, relative to the total amount of the adhesive components.

### (h) Tackifier

The patch may include a tackifier in terms of increasing the adhesive force of the adhesive layer.

In the present invention, the "tackifier" is generally a tackifier universally used in the field of patches. The tackifier is not particularly limited and may be appropriately selected in accordance with the intended purpose. Examples thereof include rosin-based resins, polyterpene-based resins, coumarone-indene resins, petroleum-based resins, terpene resins, terpene-phenol resins, and alicyclic saturated hydrocarbon resins. In terms of their actual uses in pharmaceuticals and easy availability, terpene resins, rosin-based resins, and alicyclic saturated hydrocarbon resins are preferable. These may be used alone or in combination.

In order to achieve the adhesive force necessary for obtaining sufficient medicine efficacy, the tackifier can be added. However, addition of a large amount of the tackifier decreases the drug releasing characteristics or increases the skin irritation. Therefore, the amount of the tackifier is preferably 50% by mass or less, more preferably 30% by mass or less, still more preferably 20% by mass or less, and particularly preferably 10% by mass or less, relative to the total amount of the adhesive components.

### <Other components>

The patch may include a release liner that is common in the art. That is, the patch of the present invention may include a support, an adhesive layer, and a release liner, the support, the adhesive layer, and the release liner being laminated in this order.

The release liner is not particularly limited and may be appropriately selected in accordance with the intended purpose. For example, glassine paper; a resin film such as polyolefin (e.g., polyethylene and polypropylene), polyester (e.g., polyethylene terephthalate), polystyrene, or the like; an aluminum film; an expanded polyethylene film or expanded polypropylene film; or a laminated product of two or more kinds of these materials can be used. As the release liner, for example, a release liner, which is subjected to silicone processing, fluororesin processing, emboss processing, hydrophilic processing, hydrophobic processing, or the like may also be used.

The thickness of the release liner is not particularly limited and may be appropriately selected in accordance with the intended purpose. The thickness thereof is preferably 10 µm or more and 200 µm or less, and more preferably 15 µm or more and 150 µm or less.

### (Production method of patch)

The production method of the patch, which is a method of producing the patch, is not particularly limited and is a generally performed production method such as a solvent method, a hot-melt method, or the like. For example, when the solvent method is used to produce a patch, the production method may include: spreading a coating liquid for forming adhesive layer on a release liner and drying the solvent in the coating liquid, to obtain an adhesive layer; and laminating the support and the adhesive layer.

### <Spread·drying step>

The spread-drying step is not particularly limited and may be appropriately selected in accordance with the intended purpose. Examples thereof include a method in which (a) blonanserin or a salt thereof, (b) a thermoplastic elastomer, and (c) a higher fatty acid ester are each dissolved or dispersed in a solvent such as toluene or the like, to prepare a coating liquid for forming adhesive layer, and the obtained coating liquid is coated on a release liner, followed by drying.

The coating is not particularly limited and may be appropriately selected in accordance with the intended purpose. The coating may be performed using a commonly used coater, such as a roll coater, a die coater, a gravure roll coater, a reverse roll coater, a kiss roll coater, a dip roll coater, a bar coater, a knife coater, a spray coater, or the like.

The solvent used in the coating liquid is not particularly limited and may be appropriately selected in accordance with the intended purpose. A solvent that can uniformly dissolve or disperse the (a) component, the (b) component, and the (c) component is preferable. Examples thereof include: aromatic hydrocarbons such as toluene and the like; alicyclic hydrocarbons such as cyclohexane, methylcyclohexane, and the like; aliphatic hydrocarbons such as hexane, heptane, and the like; ethers such as tetrahydrofuran, diethyl ether, t-butyl methyl ether, and the like; ketones such as acetone, methyl ethyl ketone, methyl isobutyl ketone, and the like; alcohols such as ethanol, propanol, butanol, and the like; and acetates such as ethyl acetate, propyl acetate, isopropyl acetate, butyl acetate, isobutyl acetate, and the like.

These solvents may be used alone or in combination.

In terms of good solubility of each component constituting the adhesive layer, it is more preferable to use an aromatic hydrocarbon such as toluene or the like, an alicyclic hydrocarbon such as cyclohexane, methylcyclohexane, or the like, or an aliphatic hydrocarbon such as hexane, heptane, or the like alone or as a mixture thereof, or it is more preferable to use an aromatic hydrocarbon such as toluene or the like, an aliphatic hydrocarbon such as hexane, heptane, or the like in combination with an acetate such as ethyl acetate, propyl acetate, isopropyl acetate, butyl acetate, isobutyl acetate, or the like.

The drying is not particularly limited and may be appropriately selected in accordance with the intended purpose. For example, the drying is preferably performed at temperatures of, for example, about 40°C or more and about 150°C or less under heating. The drying temperature, the drying duration, and the drying method may be adjusted in accordance with a solvent to be used or its amount.

The weight per unit area of the adhesive layer obtained after the drying may be adjusted in accordance with required adhesiveness to the skin or percutaneous absorption ability. Such a range that achieves the adhesiveness to the skin and enables the production is not particularly limited and may be appropriately selected in accordance with the intended purpose. The weight per unit area of the adhesive layer obtained after the drying is preferably 10 g/m² or more and 1,000 g/m² or less, more preferably 20 g/m² or more and 800 g/m² or less, and still more preferably 30 g/m² or more and 600 g/m² or less.

### <Lamination step>

Examples of the lamination step include a step of laminating a support.

### -Step of laminating support-

The step of laminating the support is not particularly limited and may be appropriately selected in accordance with the intended purpose. Examples thereof include a method of laminating the support and the adhesive layer through pressure bonding.

### Examples

Hereinafter, Examples of the present invention will be described, but the present invention is not limited to these Examples.

### <Examples 1 to 8: Production of patch>

According to the prescription presented in Table 1, each component constituting an adhesive layer was weighed. Note that, the numerical value of each component of Table 1 is represented by % by mass. First, after a styrene-based block copolymer was dissolved in toluene, blonanserin and octyldodecyl myristate as a higher fatty acid ester were added thereto, followed by mixing and stirring, to prepare a coating liquid for forming adhesive layer.

The coating liquid was coated on a film formed of polyethylene terephthalate (PET), which was a release liner and had been subjected to a silicone treatment, with the thickness of the coated coating liquid being adjusted so that the amount of blonanserin per 1 cm² of the adhesive layer obtained after drying was the same as that of Lonasen (Registered Trademark) tape. After the coated product was dried in an oven at 50°C for 60 minutes, the surface of the adhesive layer was laminated with a film (support) formed of PET, and was cut into a piece having a size of 15 cm × 30 cm, to obtain a patch.

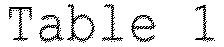

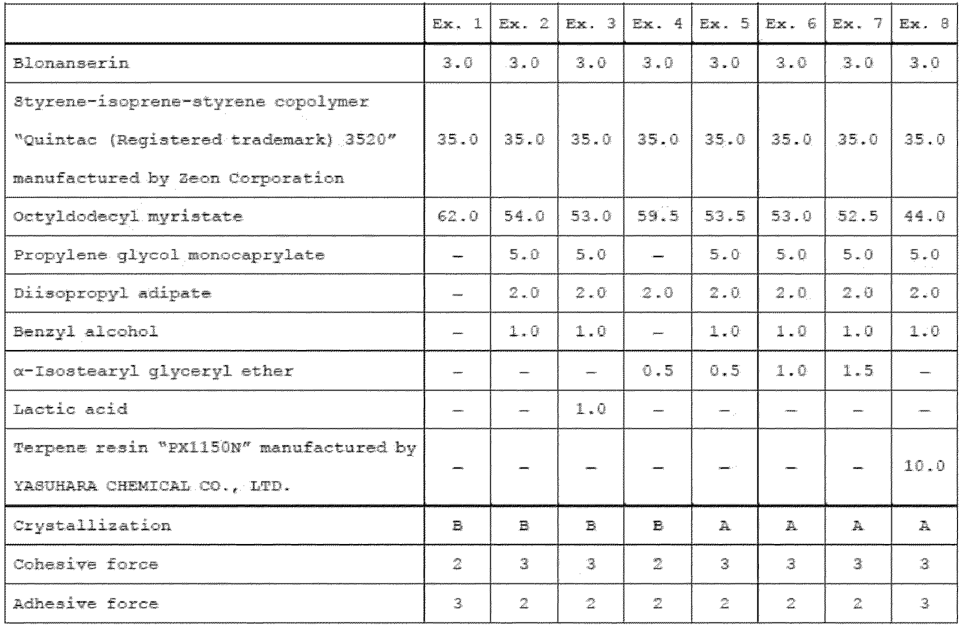

### <Test Example 1: Evaluation of crystallization>

The patches of Examples 1 to 8 were packaged with a packaging material formed of PET, and were stored at 25°C for a month. The state of crystallization of blonanserin after the storage was observed visually and microscopically. Then, the crystallization of the patch was evaluated based on the following evaluation criteria. The results are presented in Table 1.
A: No presence of crystals was visually observed at all.
B: Crystals had a particle diameter of 200 µm or less, and almost no presence of crystals was visually observed.
C: Presence of crystals was visually observed.

### <Test Example 2: Evaluation of cohesive force>

Based on the following evaluation criteria, the cohesive force of the adhesive layer of the patch was evaluated in four stages through finger tack (finger touch test) . The results are presented in Table 1.
3: No adhesive residues were observed at all.
2: Almost no adhesive residues were observed, which did not fall within a problematic rage.
1. The cohesive force was slightly lacked, but did not fall within a problematic range.
0: The adhesive residues, shape loss, and the like were observed, and the cohesive force was significantly lacked.

### <Test Example 3: Evaluation of adhesive force>

Based on the following evaluation criteria, the adhesive force of the adhesive layer of the patch was evaluated in four stages through finger tack (finger touch test) . The results are presented in Table 1.
3: The adhesive force of the adhesive layer of the patch was almost comparable to that of Lonasen (Registered Trademark) tape.
2: The adhesive force of the adhesive layer of the patch was slightly lower than Lonasen (Registered Trademark) tape.
1: The adhesive force was so weak that the adhesive layer of the patch was easily peeled off with fingers.
0: The adhesive layer of the patch was not attached at all, and its peeling was significant.

### <Comparative Examples 1 to 8: Production of patch>

A patch was produced in the same manner as in Examples 1 to 8, except that each component constituting the adhesive layer was changed to each prescription presented in Table 2. The crystallization, the cohesive force, and the adhesive force were evaluated in the same manner as in Examples 1 to 8. The results are presented in Table 2.

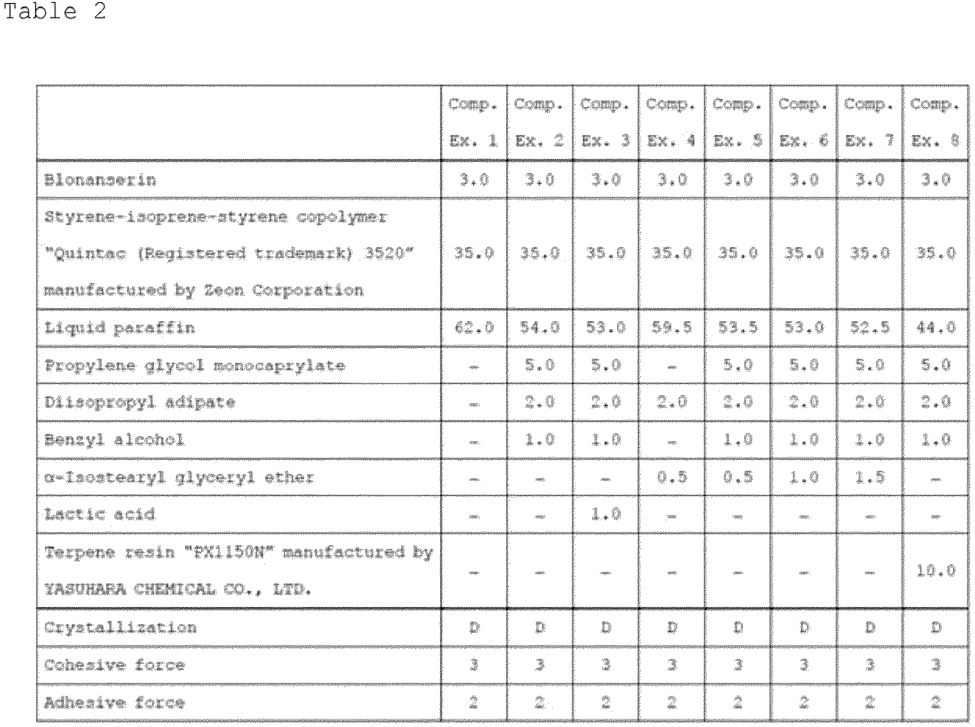

As presented in the results in Table 1, almost no crystallization of blonanserin was observed in Examples 1 to 8, in which a higher fatty acid ester was used as a plasticizer, while a phenomenon, in which blonanserin crystallized during the storage, was observed in Comparative Examples presented in Table 2. On the other hand, the adhesive force and the cohesive force of the patches of Examples were almost comparable to those of the patches of Comparative Examples.

### <Test Example 4: Evaluation of skin permeability>

The abdominal extracted skin of a hairless male rat (HWY/Slc, SPF, 5 weeks old) from which hair had been removed was immersed in a buffer solution for 30 minutes, and the moisture of the skin surface was wiped off. Then, the skin was punched out in a circle shape with a diameter of 2.5 cm. After the patch produced in Example 2 was punched out in a circle shape with a diameter of 1.3 cm and the patch was applied on the skin of the rat, it was set in a vertical diffusion cell, and the test was started using an automatic sampling apparatus for percutaneous absorption test (manufactured by CosMED Pharmaceutical Co. Ltd.). A buffer solution was used as a buffer, and the test was performed at a buffer temperature of 32°C. After 24 hours from the start of the test, a part of the buffer was sampled, the amount of the drug that had permeated the rat skin in the buffer was quantified through HPLC. As a control formulation that contains blonanserin, Lonasen (Registered Trademark) tape (traditional formulation) was used. Three cases were measured for each patch, and an average value of the measured values was calculated. The skin permeability of a drug was evaluated based on how many times the average value was relative to the value of Lonasen (Registered Trademark) tape. The results are presented in Table 3.

**Table 3**

| | Ex. 2 |
|---|---|
| Blonanserin | 3.0 |
| Styrene-isoprene-styrene copolymer "Quintac (Registered trademark) 3520" manufactured by Zeon Corporation | 35.0 |
| Octyldodecyl myristate | 54.0 |
| Propylene glycol monocaprylate | 5.0 |
| Diisopropyl adipate | 2.0 |
| Benzyl alcohol | 1.0 |
| Skin permeability of drug (times) relative to traditional formulation | 3.5 |

As presented in Table 3, the skin permeability of the patch of Example 2 was 3 times or more as high as that of the traditional formulation.

Aspects of the present invention are as follows, for example.
<1> A patch including:
   a support; and
   an adhesive layer on the support,
   wherein the adhesive layer includes blonanserin or a salt thereof, a thermoplastic elastomer, and a higher fatty acid ester, wherein the thermoplastic elastomer is a styrene-based block copolymer, and wherein a number of carbon atoms in an ester portion of the higher fatty acid ester is 10 or more and 30 or less.
<2> The patch according to <1>,
   wherein a ratio of the higher fatty acid ester relative to 100 parts by mass of the thermoplastic elastomer is 25 parts by mass or more and 300 parts by mass or less.
<3> The patch according to <1> or <2>,
   wherein an amount of the higher fatty acid ester in the adhesive layer is 75% by mass or less.
<4> The patch according to <1>,
   wherein the styrene-based block copolymer is a mixture of a styrene-isoprene-styrene block copolymer and a styrene-isoprene block copolymer.
<5> The patch according to <4>,
   wherein a ratio of the styrene-isoprene block copolymer in the mixture is 50% by mass or more.
<6> The patch according to any one of <1> to <5>,
   wherein a 25% by mass toluene solution of the styrene-based block copolymer has a viscosity of 500 mPa·s or more and 2,000 mPa·s or less at 25°C.
<7> The patch according to any one of <1> to <6>,
   wherein the adhesive layer includes an aliphatic dicarboxylic acid ester.
<8> The patch according to any one of <1> to <7>,
   wherein the adhesive layer includes glycerin monoether.
<9> A production method of the patch according to any one of <1> to <8>, the method including:
   laminating the support and the adhesive layer.

## Claims

1. A patch comprising:
a support; and
an adhesive layer on the support,
wherein the adhesive layer includes blonanserin or a salt thereof, a thermoplastic elastomer, and a higher fatty acid ester, wherein the thermoplastic elastomer is a styrene-based block copolymer, and wherein a number of carbon atoms in an ester portion of the higher fatty acid ester is 12 or more and 30 or less, or wherein the higher fatty acid ester is obtained by binding a carboxyl group of capric acid and an aliphatic alcohol via an ester bond.

2. The patch according to claim 1,
wherein a ratio of the higher fatty acid ester relative to 100 parts by mass of the thermoplastic elastomer is 25 parts by mass or more and 300 parts by mass or less.

3. The patch according to claim 1 or 2,
wherein an amount of the higher fatty acid ester in the adhesive layer is 75% by mass or less.

4. The patch according to any one of claims 1 to 3,
wherein the adhesive layer includes an aliphatic dicarboxylic acid ester.

5. The patch according to any one of claims 1 to 4,
wherein the adhesive layer includes glycerin monoether.

6. A production method of the patch according to any one of claims 1 to 5, the method comprising:
laminating the support and the adhesive layer.

## Patentansprüche

1. Pflaster umfassend:
einen Träger; und
eine Klebeschicht auf dem Träger,
wobei die Klebeschicht Blonanserin oder ein Salz davon, ein thermoplastisches Elastomer, und einen höheren Fettsäureester enthält, wobei das thermoplastische Elastomer ein Blockcopolymer auf Styrolbasis ist, und wobei die Anzahl der Kohlenstoffatome in einem Esterteil des höheren Fettsäureesters 12 oder mehr und 30 oder weniger beträgt, oder wobei der höhere Fettsäureester durch Binden einer Carboxylgruppe von Caprinsäure und eines aliphatischen Alkohols über eine Esterbindung erhalten wird.

2. Pflaster gemäß Anspruch 1,
wobei das Verhältnis des höheren Fettsäureesters relativ zu 100 Masseteilen des thermoplastischen Elastomers 25 Masseteile oder mehr und 300 Masseteile oder weniger beträgt.

3. Pflaster gemäß Anspruch 1 oder 2,
wobei die Menge des höheren Fettsäureesters in der Klebeschicht 75 Massenprozent oder weniger beträgt.

4. Pflaster gemäß mindestens einem der Ansprüche 1 bis 3, wobei die Klebeschicht einen aliphatischen Dicarbonsäureester enthält.

5. Pflaster gemäß mindestens einem der Ansprüche 1 bis 4, wobei die Klebeschicht Glycerinmonoether enthält.

6. Verfahren zur Herstellung des Pflasters gemäß mindestens einem der Ansprüche 1 bis 5, wobei das Verfahren umfasst:
Laminieren des Trägers und der Klebeschicht.

## Revendications

1. Patch comprenant :
un support ; et
une couche adhésive sur le support,
dans lequel la couche adhésive comporte de la blonansérine ou un sel de celle-ci, un élastomère thermoplastique, et un ester d'acide gras supérieur, dans lequel l'élastomère thermoplastique est un copolymère séquencé à base de styrène, et dans lequel un nombre d'atomes de carbone dans une partie ester de l'ester d'acide gras supérieur est de 12 ou plus et de 30 ou moins, ou dans lequel l'ester d'acide gras supérieur est obtenu en liant un groupe carboxyle d'acide caprique et un alcool aliphatique via une liaison ester.

2. Patch selon la revendication 1,
dans lequel un rapport de l'ester d'acide gras supérieur par rapport à 100 parties en masse de l'élastomère thermoplastique est de 25 parties en masse ou plus et de 300 parties en masse ou moins.

3. Patch selon la revendication 1 ou 2,
dans lequel une quantité de l'ester d'acide gras supérieur dans la couche adhésive est de 75 % en masse ou moins.

4. Patch selon l'une quelconque des revendications 1 à 3,
dans lequel la couche adhésive comporte un ester d'acide dicarboxylique aliphatique.

5. Patch selon l'une quelconque des revendications 1 à 4,
dans lequel la couche adhésive comporte de la glycérine monoéther.

6. Méthode de production du patch selon l'une quelconque des revendications 1 à 5, la méthode comprenant :
la stratification du support et de la couche adhésive.
